Europäisches Patentamt

European Patent Office

Office européen des brevets

(19)

(11) Numéro de publication : **0 080 647 B2**

(12) **NOUVEAU FASCICULE DE BREVET EUROPEEN**

(45) Date de publication du nouveau fascicule du brevet : **23.12.92 Bulletin 92/52**

(51) Int. Cl.⁵ : **A61F 13/15**

(21) Numéro de dépôt : **82110576.4**

(22) Date de dépôt : **16.11.82**

(54) **Couche-culotte à jeter munie de dispositifs d'attache par adhésif.**

(30) Priorité : **25.11.81 FR 8122099**

(43) Date de publication de la demande : **08.06.83 Bulletin 83/23**

(45) Mention de la délivrance du brevet : **16.10.85 Bulletin 85/42**

(45) Mention de la décision concernant l'opposition : **23.12.92 Bulletin 92/52**

(84) Etats contractants désignés : **AT BE CH DE GB IT LI**

(56) Documents cités :
**GB-A- 2 054 350**
**US-A- 3 426 756**
**US-A- 3 561 446**
**US-A- 3 616 114**

(56) Documents cités :
**US-A- 3 848 594**
**US-A- 3 867 940**
**US-A- 3 951 149**
**US-A- 4 049 001**
**US-A- 4 210 144**

(73) Titulaire : **PEAUDOUCE**
**59, Rue de la Vignette**
**F-59126 Linselles (FR)**

(72) Inventeur : **de Jonckheere, Raphael**
**797, Domaine de la Vigne**
**F-59910 Bondus (FR)**
Inventeur : **Dussaud, Jacques**
**26, avenue du Maréchal Leclerc**
**F-59110 La Madeleine (FR)**

(74) Mandataire : **Casalonga, Axel et al**
**BUREAU D.A. CASALONGA - JOSSE**
**Morassistrasse 8**
**W-8000 München 5 (DE)**

EP 0 080 647 B2

Jouve, 18, rue Saint-Denis, 75001 PARIS

## Description

La présente invention est relative à une couche-culotte à jeter utilisable pour des enfants en bas-âge ou pour des adultes incontinents, comprenant essentiellement une feuille extérieure en matière plastique imperméable, un matelas absorbant et de préférence une feuille de protection interne perméable enserrant le matelas absorbant. Les couches-culottes de ce type comportent des dispositifs d'attache par adhésif qui sont fixés par collage sur au moins la face externe de la feuille imperméable sur les bords de la partie destinée à recouvrier la partie postérieure de l'utilisateur ou partie arrière de la feuille imperméable.

Des dispositifs d'attache par adhésif pour des couches-coulottes sont bien connus (voir par exemple brevets US 3 180 355, 3 630 201, 4 047 530, 4 049 001, 4 050 453 et brevet français numéro 74 36 169). Chaque dispositif d'attache comprend généralement une languette adhésive fixée sur une partie de la feuille extérieure de la couche-culotte, notamment de la partie arrière et venant se coller, lors de la fermeture de la couche-culotte, sur l'autre partie de la feuille extérieure, notamment sur la partie avant.

Il arrive fréquemment que l'on veuille rouvrier la couche-culotte, par exemple pour vérifier si l'enfant a mouillé le matelas absorbant, et la refermer si le matelas absorbant n'est pas encore mouillé. Le même problème de réouverture et de refermeture de la couche-culotte peut se présenter lorsqu'on veut rectifier la tenue de la couche-culotte, par exemple en vue de la resserrer à la taille. Or, il s'avère qu'un telle tentative d'ouverture sur une couche-culotte à jeter sur laquelle l'enveloppe extérieure est constituée par une feuille mince provoque, soit la déchirure de la languette adhésive soit l'arrachement de la zone de la feuille à laquelle adhère la languette adhésive, cette zone arrachée de la feuille restant collée sur la languette adhésive. Dans un cas comme dans l'autre, il n'est plus possible de refermer la couche-culotte qui est ainsi inutilisable, bien que le matelas absorbant puisse ne pas encore être mouillé.

Pour permettre une telle réouverture et refermeture d'une couche-culotte à jeter, on a déjà proposé des dispositifs d'attache par adhésif (brevets US 3 989 048 et 4 014 339) dont les languettes adhésives en forme de double rabat replié en U ou en Z comprennent deux parties recouvertes d'une couche d'adhésif, ces deux parties pouvant être utilisées successivement en vue d'une première fermeture et d'une seconde fermeture de la couche-culotte. Toutefois, ces languettes à double fermeture sont de structure relativement compliquée puisqu'elles impliquent l'utilisation d'un rabat à pliage multiple, portant en plusieurs endroits bien définis des couches d'adhésif et des couches anti-adhésives. L'utilisation de ces dispositifs connus, notamment en vue de la seconde fermeture, ne présente pas non plus la simplicité requise pour ce genre d'articles et implique en particulier une déchirure du rabat en deux endroits bien déterminés.

On connaît par ailleurs un dispositif d'attache à utilisations multiples pour une couche-culotte à jeter (brevet US 4 034 752) comprenant plusieurs languettes adhésives individuelles qui sont toutes fixées de façon détachable sur une partie de la feuille extérieure recouverte d'une couche anti-adhésive; pour chaque fermeture, on détache une languette et on la colle sur les deux parties de la feuille extérieure, devant être reliées entre elles.

Dans les brevets US 3 951 149 et 4 049 001 on a décrit des dispositifs d'attache par adhésif dans lesquels une languette de fixation adhésive est munie d'un élément adhésif supplémentaire. Lors de la première utilisation du dispositif d'attache, c'est l'élément supplémentaire que vient se coller sur la face externe de la feuille imperméable dans sa partie avant. L'élément supplémentaire est recouvert sur sa face opposée d'un revêtement anti-adhésif permettant le décollage par pelage de la languette adhésive et donc l'ouverture de la couche-culotte. La refermeture de la couche-culotte se fait alors ultérieurement au moyen de la languette adhésive qui doit être à nouveau fixée sur l'élément supplémentaire précité. Pour que les décollages répétés puissent se faire sans déchirer ou endommager la face externe de la feuille imperméable, il est nécessaire de recoller chaque fois avec précision la languette adhésive à l'endroit précis où a été collé pour la première fois l'élément supplémentaire adhésif. Dans la pratique, on constate que cette exigence rend l'ouverture répétée de la couche pratiquement impossible. Par ailleurs, l'utilisation d'un tel dispositif d'attache adhésif de structure complexe est peu pratique en raison de l'existence de l'élément adhésif supplémentaire.

On connaît également par le brevet GB 2 054 350 ou le brevet US 3 867 940 l'utilisation dans des couches absorbantes à jeter munies de dispositifs d'attache par adhésif, de zones de renforcement placées à l'intérieur de la feuille imperméable délimitant la couche, afin de faciliter le décollage et le recollage ultérieur de l'attache adhésive sur la face externe de la feuille imperméable. Bien qu'une telle zone de renforcement intérieur améliore les possibilités de décollage on constate dans la pratique qu'elle n'empêche pas véritablement les risques de déchirure de feuille imperméable lorsque l'attache adhésive est décollée sans précaution.

Le brevet US 4 210 144 prévoit d'utiliser à titre de zone de renforcement, dans le même but, une pluralité de cordons d'un adhésif liquide à chaud, lesdits cordons étant disposés transversalement et écartés les uns des autres. Outre que ces cordons soient normalement disposés à l'intérieur de la feuille imperméable, entre cette dernière et le matelas absorbant, l'écartement qui subsiste entre les différents cordons ne permet pas, là non plus d'éviter tout risque de déchirure lors d'un décollage effectué sans précaution.

La présente invention a donc pour objet la réalisation d'une couche-culotte à jeter comportant un dispositif d'attache par adhésif qui puisse être aisément collé et décollé plusieurs fois sans aucune difficulté et sans risquer de déchirer ou d'endommager la feuille imperméable de la couche même lorsque l'attache adhésive est décollée rapidement et sans aucune précaution particulière.

La couche-culotte à jeter après usage selon l'invention comprend une feuille externe en matière plastique imperméable par exemple en polyéthylène, comportant une partie avant et une partie arrière. Un matelas absorbant est disposé sur la face interne de la feuille imperméable s'étendant depuis la partie avant jusqu'à la partie arrière. De préférence, une feuille de protection interne perméable par exemple réalisée en non-tissé est fixée par des lignes de collage sur des portions latérales et extrêmes de la face interne de la feuille imperméable de façon à enserrer et maintenir en position le matelas absorbant. Deux dispositifs d'attache par adhésif sont fixés par collage sur au moins la face externe de la feuille imperméable sur les bords de sa partie arrière. Chaque dispositif d'attache comporte une languette de fixation dont une portion adhésive peut être collée sur la face externe de la feuille imperméable dans sa partie avant, en vue de fermer la couche-culotte après repli du matelas absorbant entre les jambes de l'enfant. La partie avant de la feuille imperméable présente une ou plusieurs zones renforcée de façon à permettre le collage et le décollage répété des portions adhésives des languettes de fixation précitées.

Selon l'invention, la surface externe de la feuille imperméable mince de la couche-culotte est traitée de façon à présenter un aspect mat qui lui donne une apparence de matériau textile et supprime en outre tout effet de réflexion ou de crissement. Ce traitement consiste généralement en un calandrage de la feuille de matière plastique dont la surface présente alors une multitude de petites excroissances lui donnant l'aspect mat recherché. La zone renforcée est prévue sur la face externe de la feuille imperméable et la surface de ladite zone renforcée selon la présente invention, est de préférence totalement lisse par opposition à la surface traitée mat de la feuille imperméable. L'état de surface ainsi obtenu pour la zone renforcée permet d'améliorer la résistance à la traction dans son plan de la languette collée sur la zone renforcée tout en favorisant le décollage par traction selon une direction faisant un certain angle avec le plan de la languette collée. De cette manière, on obtient une excellente fixation lorsque la couche-culotte est fermée et l'on peut cependant aisément décoller par simple pelage la languette de fixation pour ouvrir la culotte.

La zone renforcée s'étend de préférence sur toute la largeur de la partie avant de la feuille imperméable. De plus, la zone renforcée présente de préférence une largeur, telle que mesurée dans le sens de l'axe longitudinal de la couche-culotte avant repli, qui soit supérieure à la largeur dans la même direction de la languette adhésive. De cette manière, il devient possible de coller et décoller à plusieurs reprises la languette adhésive dans plusieurs positions sur la partie avant de la feuille imperméable équipée de la zone renforcée.

Dans un mode de réalisation particulièrement simple, la zone renforcée est réalisée par collage sur la face externe de la partie avant de la feuille imperméable d'une bande de matière plastique, par exemple de polypropylène, dont la surface extérieure est parfaitement lisse.

On comprendra que l'invention puisse être appliquée à tous les types de dispositifs d'attache par adhésif. C'est ainsi que l'invention peut être utilisée avec un dispositif d'attache dans lequel la languette de fixation adhésive est repliée et collée de façon provisoire sur un élément d'ancrage fixé par collage sur le bord de la face interne de la feuille imperméable.

L'invention peut également être appliquée au cas où le dispositif d'attache par adhésif comporte un élément de protection recouvrant la portion adhésive de la languette de fixation et destiné à être détaché par pelage au moment de l'utilisation.

L'invention sera mieux comprise à l'étude de quelques modes de réalisation décrits à titre d'exemples nullement limitatifs et illustrés par les dessins annexés, sur lesquels:

la fig. 1 est une vue en plan d'une couche-culotte à jeter selon l'invention;

la fig. 2 est une vue en coupe selon II II de la fig. 1 montrant un dispositif d'attache par adhésif avant utilisation;

la fig. 3 est une double vue en coupe dont une partie est prise selon III III de la fig. 1 et qui montre le dispositif d'attache par adhésif prêt à être utilisé;

la fig. 4 est une vue en perspective schématique montrant la couche-culotte après fermeture;

la fig. 5 est une vue en coupe partielle d'un autre dispositif d'attache par adhésif pouvant être utilisé dans la présente invention; et

la fig. 6 est une vue en coupe partielle d'un autre mode de réalisation d'un dispositif d'attache par adhésif pouvant être utilisé dans l'invention.

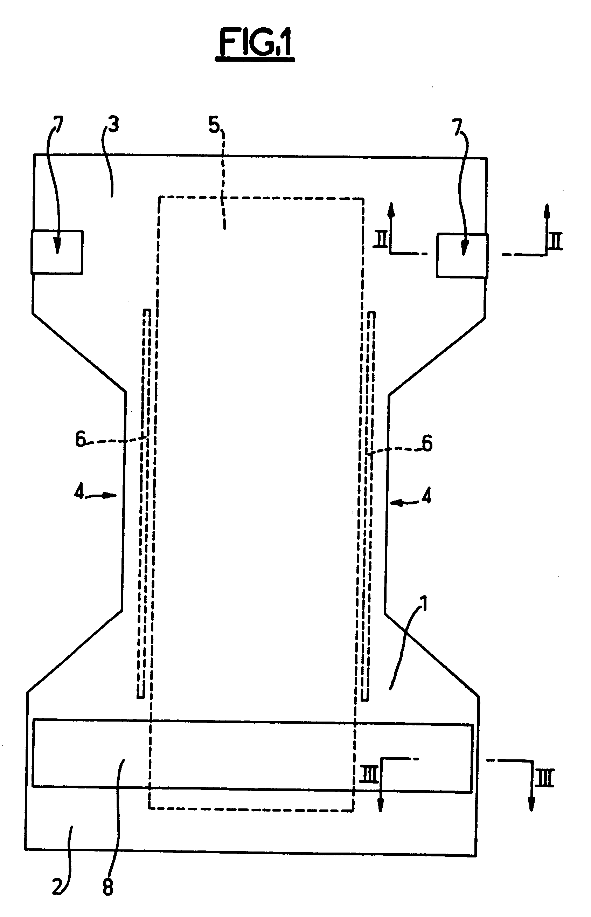

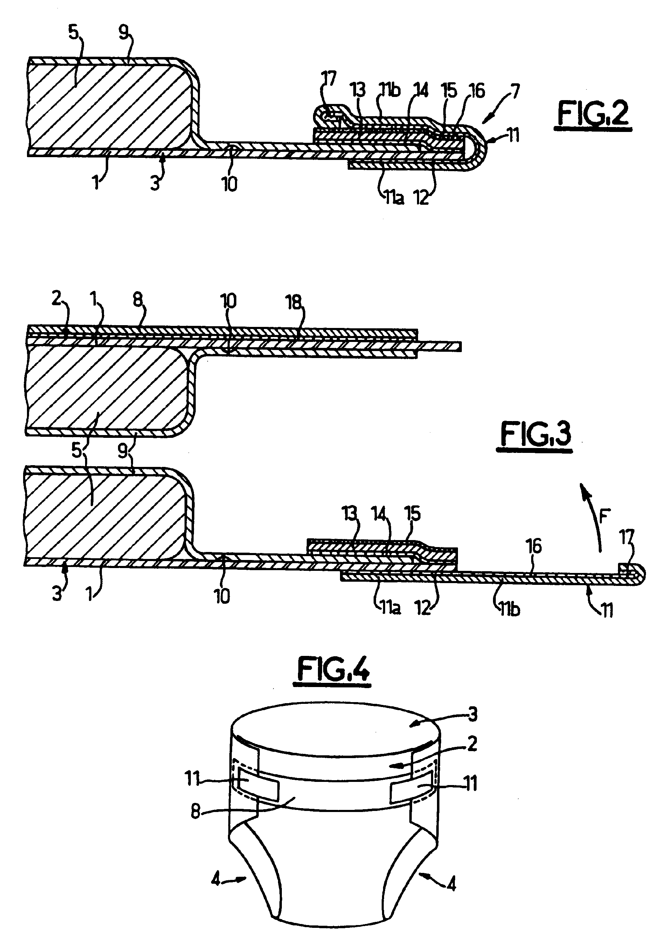

Telle qu'elle est représentée selon la fig. 1, la couche-culotte de l'invention comprend une feuille externe 1 réalisée en matière plastique imperméable mince et dont la surface extérieure est traitée de façon à lui conférer un aspect mat. La feuille imperméable 1 est découpée de façon à définir une partie avant 2 et une partie arrière 3 qui sont séparées selon l'axe longitudinal de la couche-culotte par des échancrures 4 destinées à former les passages des jambes visibles en particulier sur la fig. 4. Un matelas absorbant 5 est placé sur la face intérieure de la feuille imperméable 1 selon l'axe longitudinal de la couche-culotte, les bords longitudinaux du matelas 5 étant proches et parallèles des bords des échancrures 4 tandis que les bords transversaux du matelas 5 sont disposés à une certaine distance des bords extrêmes des parties avant et arrière 2 et 3. Des élastiques 6 sont disposés, dans ce mode de réalisation, au voisinage immédiat des bords longitudinaux du matelas 5 sur la face interne de la feuille imperméable 1 et parallèlement à l'axe longitudinal dans la zone de l'entrejambe.

Deux dispositifs d'attache par adhésif 7 sont fixés sur les bords latéraux de la partie arrière 3 sensiblement au niveau de l'un des bords transversaux du matelas 5.

Tel qu'illustré sur la fig. 1 une bande de matière plastique 8 de surface extérieure lisse est collée transversalement sur toute la largeur de la partie avant 2 de la feuille imperméable 1 sur la face externe de cette dernière. La largeur de la bande 8 est sensiblement égale à 1,5 fois la largeur des dispositifs d'attache 7, telle que mesurée selon l'axe longitudinal de la couche-culotte.

On se reportera maintenant aux fig. 2 et 3 qui montrent schématiquement en version très agrandie la coupe d'un mode de réalisation du dispositif d'attache par adhésif qui peut être utilisé dans la présente invention. Comme on peut le voir sur les fig. 2 et 3 où les éléments identiques portent les mêmes références, une feuille de protection interne perméable 9 se trouve fixée par des lignes de colle 10 sur les bords latéraux de la face interne de la feuille imperméable 1 de façon à enserrer et maintenir en position le matelas absorbant 5. La feuille de protection 9 est de préférence réalisée en non-tissé.

Le dispositif d'attache par adhésif 7 comporte une languette de fixation 11 qui est collée par une portion de fixation 11 a sur la face externe de la feuille imperméable 1 au moyen d'une couche d'adhésif 12. Un élément d'ancrage 13 est fixé par collage sur le bord de la face interne de la feuille imperméable 1 et de la feuille perméable 9 au moyen de la couche d'adhésif 14 qui assure une fixation indéchirable. Sur sa face opposée ou face externe, l'élément d'ancrage 13 présente une couche anti-adhésive 15 qui peut être à base de silicone. Sur cette couche anti-adhésive 15 vient se coller de manière provisoire la portion adhésive 11b de la languette de fixation 11 qui présente une couche d'adhésif 16 et qui se trouve repliée comme on peut le voir sur la fig. 2. Le bord extrême présente également un repli 17 facilitant la préhension. Pour utiliser le dispositif d'attache par adhésif représenté sur la fig. 2, il suffit donc de saisir le repli 17 qui n'adhère pas sur l'élément d'ancrage 13 et de détacher la portion adhésive 11b de la languette de fixation par pelage ce qui est rendu possible en raison de la couche anti-adhésive 15 de l'élément d'ancrage 13. La languette de fixation se trouve alors dans la position illustrée à la partie inférieure de la fig. 3. On a représenté à la partie supérieure de cette même fig. 3 la coupe de la partie avant de la couche-culotte selon III III de la fig. 1. On retrouve sur cette coupe la bande de matière plastique 8 jouant le rôle de zone de renforcement fixée sur la face externe de la feuille imperméable 1 par l'intermédiaire de la couche d'adhésif 18. Pour fermer la couche-culotte il suffit donc de saisir la languette de fixation 11 et de venir la fixer sur la bande de renforcement 8 en faisant le mouvement schématisé sur la fig. 3 par la flèche F.

La couche-culotte une fois fermée présente l'aspect illustré sur la fig. 4. On voit que les deux languettes de fixation 11 se trouvent fixées par collage sur la bande de matière plastique de renforcement 8. Grâce à l'existence de ce renfort il devient possible de décoller par pelage les languettes de fixation 11 sans risquer le déchirement ou la détérioration de la feuille imperméable 1. Cet effet est obtenu notamment en raison de la surface extérieure de la bande de matière plastique de renforcement 8 qui est parfaitement lisse. Dans la pratique, on pourra utiliser une bande en polypropylène pour obtenir ce résultat.

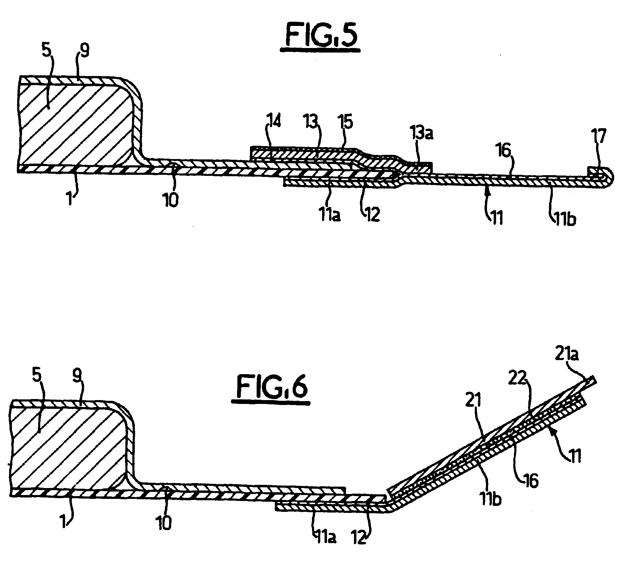

Les fig. 5 et 6 illustrent deux autres variantes non limitative de dispositifs d'attache par adhésif qui peuvent être utilisés dans le cadre de la présente invention.

Dans la variante de la fig. 5 analogue à celle des fig. 2 et 3, le dispositif d'attache est représenté en position ouverte, prêt à être utilisé. On retrouve comme dans le mode de réalisation des fig. 2 et 3, un élément d'ancrage 13 qui comprend toutefois une partie en saillie 13a qui déborde à l'extérieur du bord de la feuille imperméable 1 et vient se coller sur la face encollée 12 de la languette de fixation 11. Dans ce mode de réalisation, la traction de la languette de fixation 11 s'exerce donc sur les deux faces de la feuille imperméable 1 par l'intermédiaire d'une part de la portion II a et d'autre part de l'élément d'ancrage 13.

Dans le mode de réalisation de la fig. 6, un élément de protection 21 recouvre la portion adhésive 11b de la languette de fixatin 11. L'élément de protection présente sur sa face en regard de la languette 11 une couche anti-adhésive 22 réalisée par exemple à base de silicone. De plus, l'élément de protection 21 présente un bord 21a non collé faisant saillie à l'extérieur de l'extrémité de la languette de fixation 11. Pour utiliser un tel dispositif d'attache, il suffit donc de détacher par pelage l'élément de protection 21 en le saisissant par son extrémité 21a.

Dans la description les expressions « attache par adhésif » doivent être comprises comme signifiant un moyen de fixation qui peut être collé par pression sur un support. Les expressions « couche anti-adhésive » recouvrent des revêtements de produits susceptibles de réduire le caractère adhésif des moyens de fixation par pression en faicilitant le décollage par traction dans une direction faisant unangle avec le plan de fixation, c'est-à-dire par « pelage ».

L'homme de l'art connaît parfaitement des différents produits de revêtement qu'il est possible d'utiliser pour obtenir ces résultats.

Il y a lieu de noter que l'invention s'applique à tous les types de couches-culottes, notamment avec ou sans élastiques p la taille et/ou à l'entrejambe, avec matelas absorbant de toutes formes s'étendant jusqu'aux bords latéraux de la feuille extérieure ou se terminant à distance de ces bords, avec ou sans échancrures pour les jambes, etc.

## Revendications

1. Couche-culotte à jeter après usage comprenant une feuille externe en matière plastique imperméable (1) dont la surface externe est traitée de façon à présenter un aspect mat, comportant une partie avant (2) et une partie arrière (3), un matelas absorbant (5) disposé sur la face interne de la feuille imperméable (1), une feuille de protection interne perméable (9) fixée par collage sur des portions de la face interne de la feuille imperméable (1) et enserrant le matelas absorbant (5) et deux dispositifs d'attache par adhésif (7) fixés par collage sur au moins la face externe de la feuille imperméable (1) sur les bords de sa partie arrière (3), chaque dispositif d'attache comportant une languette de fixation (11) dont une portion (11b) munie d'une couche adhésive (16) sensible à la pression peut être collée sur la face externe de la feuille imperméable (1) dans sa partie avant (2) en vue de fermer la couche-culotte, la partie avant (2) de la feuille imperméable (1) présentant une ou plusieurs zone renforcée de façon à permettre le collage et le décollage répété des portions adhésives (11b) des languettes précitées, caractérisée par le fait que chaque zone renforcée comprend une bande de matière plastique ayant une surface extérieure lisse, fixée transversalement sur la face externe de la partie avant de la feuille imperméable, de façon à améliorer la résistance à la traction dans son plan de la languette (11) collée sur la zone renforcée tout en favorisant le décollage par traction selon une direction faisant un angle avec son plan.

2. Couche-culotte selon la revendication 1, caractérisée par le fait que la zone renforcée s'étend sur toute la largeur de la partie avant (2) de la feuille imperméable (1).

3. Couche-culotte selon l'une des revendications précédentes, caractérisée par le fait que la zone renforcée présente une largeur supérieure à celle de la languette adhésive (11) telle que mesurée dans le sens longitudinal de la couche-culotte.

4. Couche-culotte selon l'une quelconque des revendications précédentes, caractérisée par le fait que la bande de matière plastique est fixée par collage.

5. Couche-culotte selon l'une quelconque des revendications précédentes, caractérisée par le fait que chaque dispositif comporte un élément d'ancrage (13) fixé par collage sur le bord de la face interne de la feuille imperméable (1) et muni sur sa face externe d'une couche anti-adhésive (15) sur laquelle la portion adhésive (11b) de la languette de fixation (11) peut être repliée et collée de façon provisoire afin d'être détachée par pelage au moment de l'utilisation.

6. Couche-culotte selon l'une quelconque des revendications précédentes, caractérisée par le fait que lélément d'ancrage (13) déborde à l'extérieur de la feuille imperméable (1), une partie en saillie (13a) venant se coller sur la face encollée (12) de la languette de fixation (11).

7. Couche-culotte selon l'une quelconque des revendications 1 à 4, caractérisée par le fait que chaque dispositif d'attache comporte un élément de protection (21) recouvrant la portion adhésive (11b) de la languette de fixation (11) et muni sur sa face enregard de ladite languette d'une couche anti-adhésive (22) pour être collée de façon provisoire en vue d'être détachée par pelage au moment de l'utilisation.

8. Couche-culotte selon la revendication 4, caractérisée par le fait que la bande de matière plastique est réalisée en polypropylène.

9. Couche-culotte selon l'une quelconque des revendications précédentes, caractérisée par le fait qu'elle comprend des éléments élastiques disposés le long des bords longitudinaux du matelas absorbant sur la surface interne de la feuille imperméable.

10. Couche-culotte selon la revendication 9, caractérisée par le fait que les éléments élastiques sont parallèles à l'axe longitudinal de la couche-culotte.

## Patentansprüche

1. Wegwerfwindel, mit einer Außenlage aus undurchlässigem Kunststoff (1), deren Außenfläche so behandelt ist, daß sie ein mattes Aussehen hat, die einen vorderen Teil (2) und einen hinteren Teil (3) besitzt, mit einem saugfähigen Polster (5), der auf der Innenfläche der undurchlässigen Lage (1) angeordnet ist, mit einer inneren durchlässigen Schutzlage (9), die durch Kleben auf Teilen der Innenfläche der undurchlässigen Lage (1) befestigt ist und die den saugfähigen Polster (5) einschließt, und mit zwei Klebeverschlüssen (7), die durch Kleben auf wenigstens der Außenfläche der undurchlässigen Lage (1) an den Rändern ihres hinteren Teils (3) befestigt sind, wobei jeder Verschluß eine Befestigungszunge (11) besitzt, von der ein Teil (11b), der mit einer Lage eines druckempfindlichen Klebstoffes (16) versehen ist, zum Schließen der Windel auf die Außenfläche der undurchlässigen Lage (1) an ihrem vorderen Teil (2) angeklebt werden kann, wobei der vordere Teil (2) der undurchlässigen Lage (1) einen oder mehrere verstärkte Bereiche aufweist, um das wiederholte Ankleben und Lösen der Klebebereiche (11b) der vorerwähnten Zungen zu gestatten, dadurch gekennzeichnet, daß jede verstärkte Zone ein Kunststoffband mit einer glatten Außenfläche aufweist, das quer auf der Außenfläche des vorderen Teils der undurchlässigen Lage befestigt ist, um die Zugfestigkeit der auf die verstärkte Zone angeklebten Zunge (11) in ihrer Ebene zu verbessern, obwohl zugleich das Ablösen durch Zug in einer Richtung, die mit ihrer Ebene einen Winkel einschließt, erleichtert ist.

2. Windel nach Anspruch 1, dadurch gekennzeichnet, daß sich die verstärkte Zone über die ganze Breite des Vorderteils (2) der undurchlässigen Lage (1) erstreckt.

3. Windel nach einem der vorhergehenden Ansprüche, dadurch gekennzeichnet, daß die verstärkte Zone eine Breite aufweist, die größer ist als die in Längsrichtung der Windel gemessene der Klebezunge (11).

4. Windel nach einem der vorhergehenden Ansprüche, dadurch gekennzeichnet, daß das Kunststoffband durch Kleben befestigt ist.

5. Windel nach einem der vorhergehenden Ansprüche, dadurch gekennzeichnet, daß jede Anordnung ein Verankerungselement (13) aufweist, das durch Kleben am Rand der Innenseite der undurchlässigen Lage (1) befestigt ist und das auf seiner Außenseite mit einer antiadhäsiven Lage (15) versehen ist, auf welche der klebende Bereich (11b) der Befestigungszunge (11) umgeschlagen und vorübergehend angeklebt werden kann, um im Zeitpunkt der Verwendung durch Abziehen abgelöst zu werden.

6. Windel nach einem der vorhergehenden Ansprüche, dadurch gekennzeichnet, daß das Verankerungselement (13) über das Äußere der undurchlässigen Lage (1) mit einem vorspringenden Teil (13a) hinausragt, der auf der mit Klebstoff versehenen Fläche (12) der Befestigungszunge (11) haftet.

7. Windel nach einem der Ansprüche 1 bis 4, dadurch gekennzeichnet, daß jeder Verschluß ein Schutzelement (21) aufweist, das den klebenden Bereich (11b) der Befestigungszunge (11) überdeckt, und der auf seiner der Zunge zugekehrten Seite mit einer antiadhäsiven Lage (22) versehen ist, der vorläufig festgeklebt werden kann, um im Zeitpunkt der Verwendung durch Abziehen abgelöst zu werden.

8. Windel nach Anspruch 4, dadurch gekennzeichnet, daß das Band aus Kunststoff aus Polypropylen hergestellt ist.

9. Windel nach einem der vorhergehenden Ansprüche, dadurch gekennzeichnet, daß sie entlang der Längsränder des absorbierenden Polsters auf der Innenseite der undurchlässigen Lage elastische Elemente aufweist.

10. Windel nach Anspruch 9, dadurch gekennzeichnet, daß die elastischen Elemente zur Längsachse der Windel parallel sind.

## Claims

1. Nappy-pants for disposal after use, comprising an outer sheet made of impermeable plastic (1) of which the outer surface is treated so as to have a matt appearance, incorporating a front part (2) and a back part (3), an absorbent pad (5) arranged on the inner face of the impermeable sheet (1) a permeable inner protective sheet (9) fastened by gluing to parts of the inner face of the impermeable sheet (1) and firmly enclosing the absorbent pad (5) and two devices for attachment by an adhesive (7) which are fastened gluing to at least the outer face of the impermeable sheet (1) to the edges of its back part (3), each device incorporating a fastening tab (11), a portion of which (11b), equipped with a pressure-sensitive adhesive layer (16), may be glued to the outer face of the impermeable sheet (1) in its front part (2) in order to close the nappy-pants, the front part (2) of the impermeable sheet (1) having one or more strengthened zones so as to allow the repeated gluing and ungluing of the adhesive portions (11b) of the abovementioned tabs, characterised in that each strengthened zone comprises a strip of plastic having a smooth outer surface and fastened transversely to the outer surface of the front part of the impermeable sheet, so as to improve the tensile strength in its plane of the tab (11) glued to the strengthened zone, while promoting the ungluing by pulling in a direction forming an angle with its plane.

2. Nappy-pants according to Claim 1, characterised in that the strengthened zone extends over the entire width of the front part (2) of the impermeable sheet (1).

3. Nappy-pants according to one of the preceding claims, characterised in that the strengthened zone has a width greater than that of the adhesive tab (11) as measured in the lengthwise direction of the nappy-pants.

4. Nappy-pants according to any one of the preceding claims, characterised in that the strip of plastic (8) is fastened by gluing.

5. Nappy-pants according to any one of the preceding claims, characterised in that each device incorporates an anchoring member (13) fastened by gluing to the edge of the inner face of the impermeable sheet (1) and equipped on its outer face with an anti-adhesive layer (15) onto which the adhesive portion (11b) of the fastening tab (11) may be folded back and glued in a provisional manner, in order to be pulled off by peeling at the time of use.

6. Nappy-pants according to any one of the preceding claims, characterised in that the anchoring member (13) extends outside the impermeable sheet (1), a projecting part (13a) being made to be glued to the glue-coated face (12) of the fastening tab (11).

7. Nappy-pants according to any one of Claims 1 to 4, characterised in that each attachment device incorporates a protective member (21) covering the adhesive portion (11b) of the fastening tab (11) and equipped on the face facing the said tab with an anti-adhesive layer (22) for being glued in a provisional manner in order to be pulled off by peeling at the time of use.

8. Nappy-pants according to Claim 4, characterised in that the strip of plastic is made of polypropylene.

9. Nappy-pants according to any one of the preceding claims, characterised in that they comprise elastic members arranged along the lengthwise edges of the absorbent pad on the inner surface of the impermeable sheet.

10. Nappy-pants according to Claim 9, characterised in that the elastic members are parallel to the lengthwise axis of the nappy-pants.

# FIG.1

FIG.2

FIG.3

FIG.4

## FIG.5

## FIG.6